# EUROPEAN PATENT SPECIFICATION

(11) **EP 1 648 440 B1**
(45) Date of publication and mention of the grant of the patent: **12.03.2008**
(21) Application number: 04741331.5
(22) Date of filing: 15.07.2004
(51) Int. Cl.: A61K 31/337, A61K 9/48

(54) **SEMI-SOLID FORMULATIONS FOR THE ORAL ADMINISTRATION OF TAXOIDS**
HALBFESTE FORMULIERUNG FÜR DIE ORALE VERABREICHUNG DES TAXOLS
FORMULATION SEMI-SOLIDE POUR L'ADMINISTRATION ORALE DU TAXOL

(30) Priority: 18.07.2003 EP 03291795
(43) Date of publication of application: 26.04.2006
(73) Proprietor: Aventis Pharma S.A., 92160 Antony (FR)
(72) Inventor: BOROVAC, Tatiana, F-92120 Montrouge (FR); NEVES, Carole, F-75012 Paris (FR); PERACCHIA, Maria-Teresa, F-75005 Paris (FR)
(74) Representative: Le Pennec, Magali
(86) International application number: PCT/EP2004/008551
(87) International publication number: WO 2005/013968

(56) References cited:
- WO-A-01/30319
- WO-A-99/49848
- WO-A-02/064132
- WO-A-03/045357
- WO-A-03/074027
- MU L ET AL: "A novel controlled release formulation for the anticancer drug paclitaxel (Taxol<(>R)): PLGA nanoparticles containing vitamin E TPGS" JOURNAL OF CONTROLLED RELEASE, ELSEVIER SCIENCE PUBLISHERS B.V. AMSTERDAM, NL, vol. 86, no. 1, 9 January 2003 (2003-01-09), pages 33-48, XP004398997 ISSN: 0168-3659
- ADAMS M W: "D-ALPHA TOCOPHERYL POLYETHYLENE GLYCOL 1000 SUCCINATE (EASTMAN VITAMI E TPGS) AS AN EMULSIFIER AND BIOENHANCER FOR DRUGS AND LIPOPHILIC COMPOUNDS" INTERNATIONAL CONGRESS OF TECHNOLOGY AND PHARMACOLOGY, ASSOC. PHARM. GALENIQUE IND., CHATENAY MALABRY, FR, 1992, pages 254-262, XP000576964

## Description

The present invention relates to oral formulations of taxoids.

The taxoids used in the formulations according to the invention are preferably of the general formula (I) 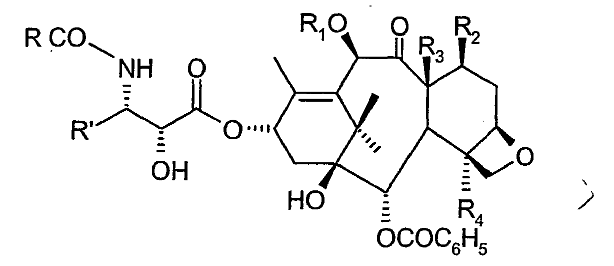 wherein
R₁ is H, acyl (C₂-C₄), alkyl (C₁-C₃),
R₂ is OH, alkoxy or R₂ and R₃ are methylene,
R₃ is CH₃ or R₂ and R₃ are methylene,
R₄ is OCOCH₃ or OCOOCH₃,
R is phenyl or alkoxy (C₃-C₄) or alkenyloxy (C₃-C₄), preferably phenyl or tert-butoxy,
R' is aryl, preferably phenyl, optionally substituted or alkyl (C₂-C₄) or alkylen (C₂-C₄).

The taxoids used in the formulations according to the invention are for example the taxoids of formula (Ia) to (If) below

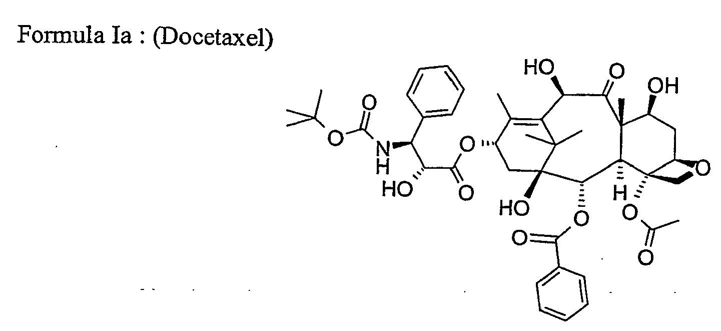

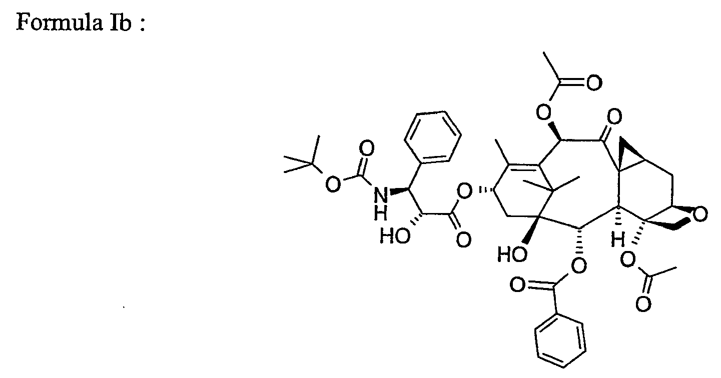

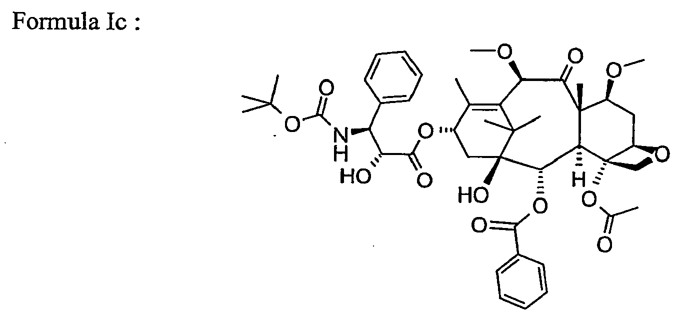

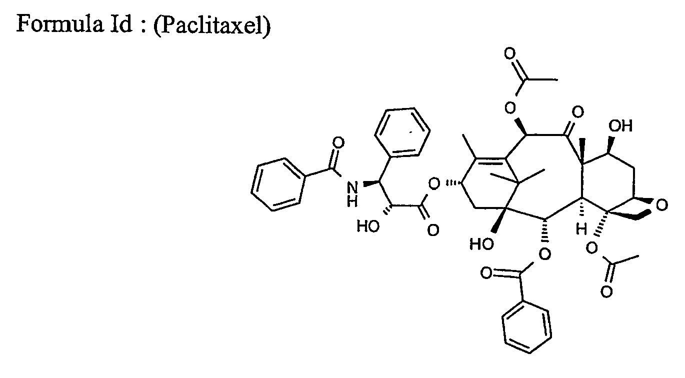

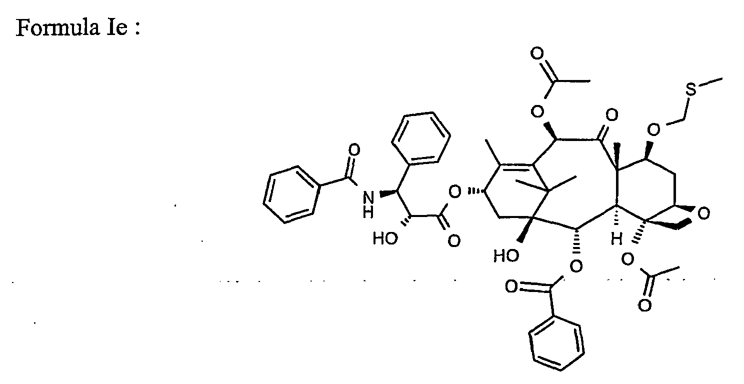

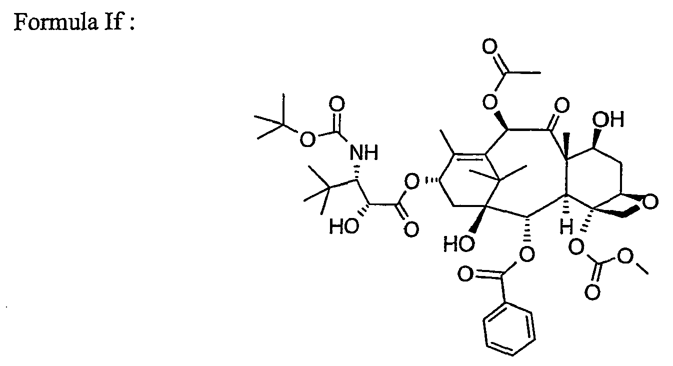

Taxoids of general formula (Ia) to (If) and their applications are known. These taxoids are particularly advantageous for their use as chemotherapeutic agents.

Unfortunately, taxoids are poorly water-soluble compounds. The molecules are slightly lipophilic with a relatively high molecular weight. Up until now taxoids are administered intravenously, in particular using formulations consisting of PS80 or cremophor at high content. It was the aim of the current invention to develop taxoid formulations for oral administration.

Oral administration of PS80 or cremophor formulations of taxoids led to an extremely low bioavailability in animals probably because of a high metabolism rate, like e.g. dogs. In addition, formulations consisting of a high content of PS80 (e.g. less than 40 mg taxoid/g PS80) are not desirable for oral administration because of the potential toxicity of PS80 in contact with the intestinal mucosa. Furthermore, a dose escalation study would not be possible with the expected doses because of the solubility limit and as a consequence the limited PS80 solubilisation capacity for taxoids in gastro-intestinal fluids. Finally, the pharmaceutical development of a drug dosage form would be a main issue: indeed, the extemporary dilution of the PS80 solution with an aqueous medium is not envisageable for the oral administration of a cytotoxic agent.

Numerous documents describe systems suitable for solubilising and/or enhancing the bioavailability of hydrophobic active ingredients. However, the systems tested have so far proved ineffective for the preparation of pharmaceutical compositions containing taxoids which are stable and bioavailable and in which the taxoid can be administered orally at an effective concentration.

WO 95/24893 describes delivery systems for hydrophobic drugs. This application describes compositions comprising a digestible oil, a lipophilic surfactant and a hydrophilic surfactant that are intended for the formulation of hydrophobic active ingredients and for the enhancement of their bioavailability.

WO 99/49848 describes pharmaceutical dosage forms for anticancer drugs, e.g. paclitaxel in which the active drug is formulated as stable self-emulsifying preconcentrate. WO 99/49848 describes compositions comprising an anticancer drug in a carrier system comprising at least one hydrophobic component selected from tri-, di- or monoglycerides, free fatty acids, fatty acid esters or derivatives thereof, and a hydrophilic component selected from hydroxyalkane, dihydroxyalkane or polyethylene glycol (PEG), and comprising at least one surfactant.

EP 0 152 945 B1 describes transparent multi-component systems for pharmaceutical application containing one or several active ingredients in a system composed of an oil component, surfactants, co-surfactant and optionally water.

EP 0 670 715 B1 describes compositions for pharmaceutical use intended to be ingested, able to form a microemulsion, comprising at least an active ingredient, a lipophilic phase, a surfactant, a co-surfactant and a hydrophilic phase of special composition.

EP 0 334 777 B1 describes a micro-emulsion with pharmaceutical use comprising a water-soluble phase and a lipidic phase, comprising at least one surface-active agent based on Polyethylenglycol and at least one co-surfactant based on polyglycerol.

It has now been found, and that is what constitutes the subject of the present invention, that it is possible to prepare chemically and physically stable formulations of taxoid for oral administration. The present invention relates to a semi-solid formulation for the oral administration of taxoids consisting of one taxoid and Vitamin E TPGS®.

Preferably, the taxoid is of general formula (I) : 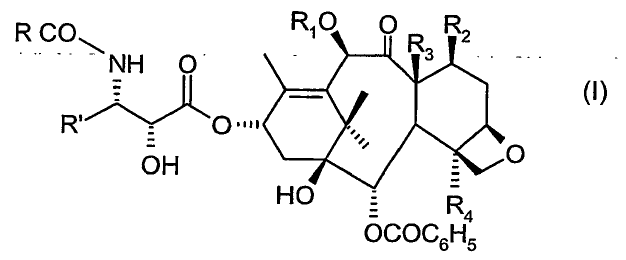 wherein :
R₁ is H, acyl (C₂-C₄), alkyl (C₁-C₃);
R₂ is OH, alkoxy or R₂ and R₃ are methylene;
R₃ is CH₃ or R₂ and R₃ are methylene;
R₄ is OCOCH₃ or OCOOCH₃;
R is phenyl or alkoxy (C₃-C₄) or alkenyloxy (C₃-C₄), preferably phenyl or tert-butoxy; and
R' is aryl, preferably phenyl, optionally substituted or alkyl (C₂-C₄) or alkylen (C₂-C₄).

A more preferred taxoid is chosen among compounds of formula (Ia) to (If) :

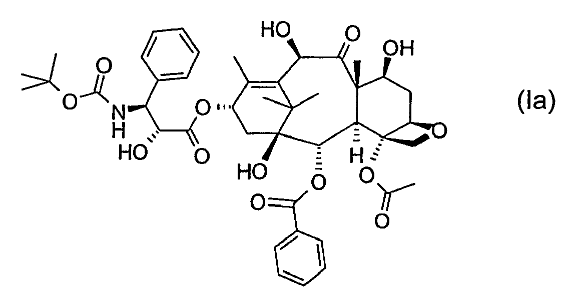

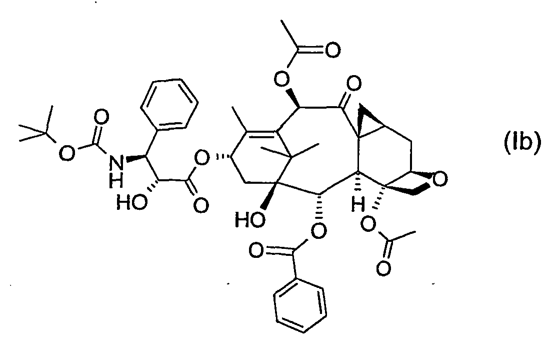

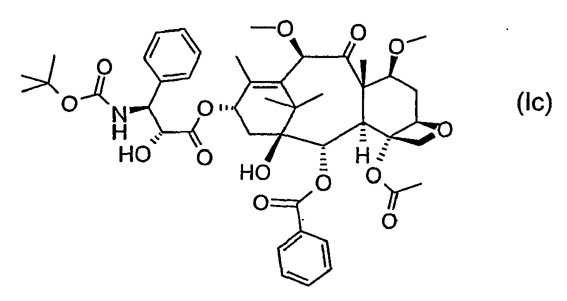

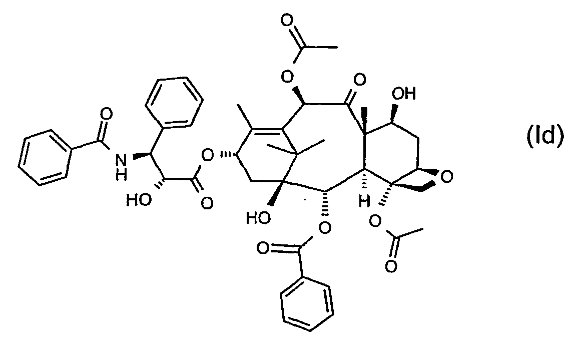

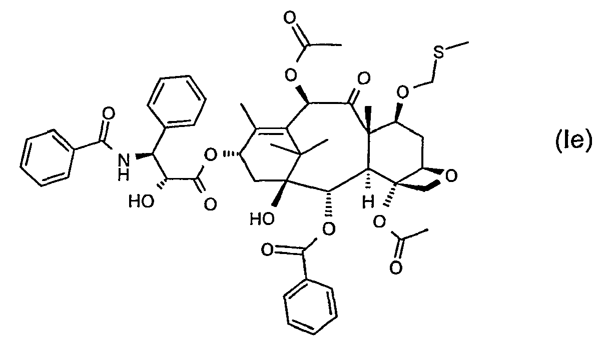

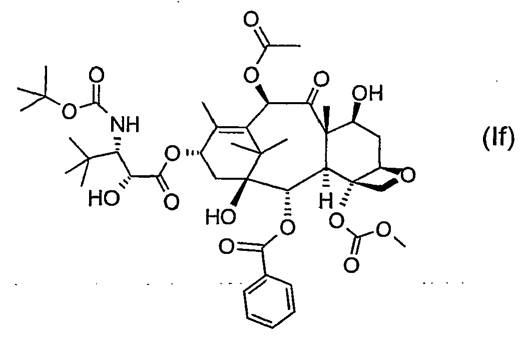

The semi-solid formulation of the invention is particularly suitable for taxoids of formula (Ib) and (Ic).

A convenient semi-solid formulation according to the invention may contain up to 200 mg taxoid per g of polymeric material, more preferably between 50 and 200 mg taxoid per g of polymeric material. Suitable taxoid content may be adapted to the need of a patient, for example taxoid concentration within the polymeric material of e.g. 5 mg/g, 10 mg/g, 20 mg/g, 30 mg/g, 40 mg/g, 50 mg/g, 60 mg/g, 70 mg/g, 80 mg/g, 90 mg/g, 100 mg/g, 150 mg/g or 200 mg/g.

In another aspect the invention concerns a process for preparing a formulation as defined above, wherein there is prepared, where appropriate, the mixture of principal excipient, after heating, for melting the semisolid excipient, and then the taxoid and stirring is maintained in order to obtain a homogeneous mixture.

The strategy has been to obtain a formulation able to enhance taxoid solubilisation in aqueous medium by using amphiphilic- and lipid-based formulations able to form a colloidal system (fine emulsion or micellar solution) *in vivo.*

Among amphiphilic and lipid-based formulations, 3 categories were identified:
Amphiphilic polymers (micelle or emulsion formation)
Phospholipids (lipidic vesicles formation)
SMES (self-microemulsifying systems): oil + surfactant + co-surfactant (microemulsion formation)

After a first selection of proper excipients (in terms of safety and developpability), the solubility of taxoids in the excipient was the first screening step for the choice of the excipient and the selection of the prototypes. Then, the prototypes (liquid or semi-solid) were manufactured, and characterized in terms of *in vitro* behaviour in simulated GI media and chemical stability. Finally, the physical properties and stability of the semi-solid prototypes have been investigated.

Different categories of excipients described in the literature as components of amphiphilic and lipid-based formulations have been tested for the solubility of taxoids:
1. Oils (medium-chain triglycerides, fatty acids, ...)
2. Amphiphilic surfactants with hydrophilic character (HLB>10) (PEO sorbitan fatty acids, castor oil ethoxylates, fatty acid ethoxylates.)
3. Amphiphilic surfactants with lipophilic character (HLB<10) (glycerides of fatty acids: glyceryl oleate/linoleate, oleoyl macrogol glycerides; derivatives of propylene glycol: PG caprylate/linoleate,)
4. Phospholipids (lecithins)
5. Hydrophilic solvents (PEG 400,..)

All the selected excipients are described as safe for oral administration, and they are developable (alone or as mixture) as pharmaceutical dosage form (soft or hard capsule).

The chemical composition of the selected excipients in liquid form at room temperature, as well as the solubility of taxoid of formula Ib, are reported in table 1 below.

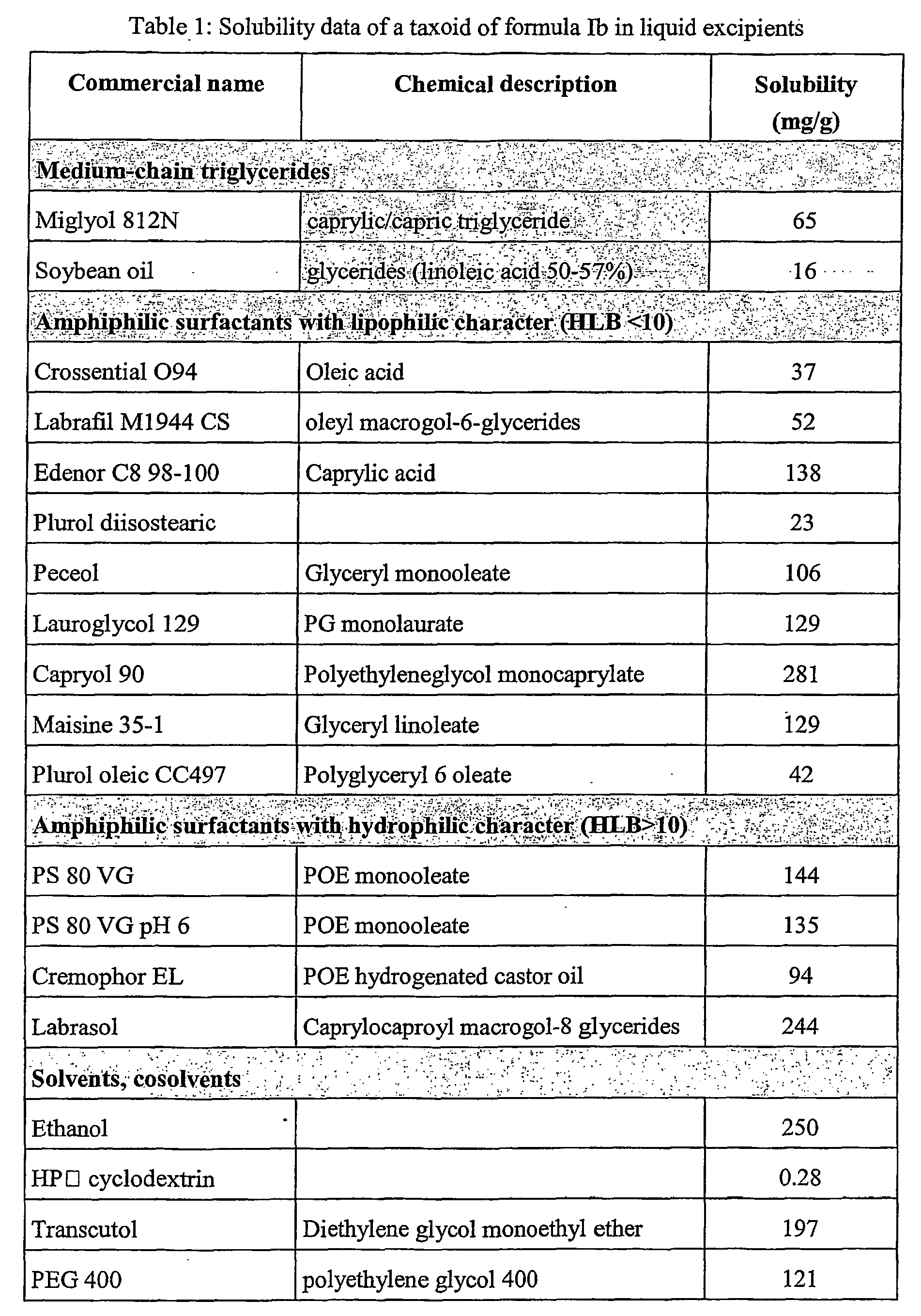

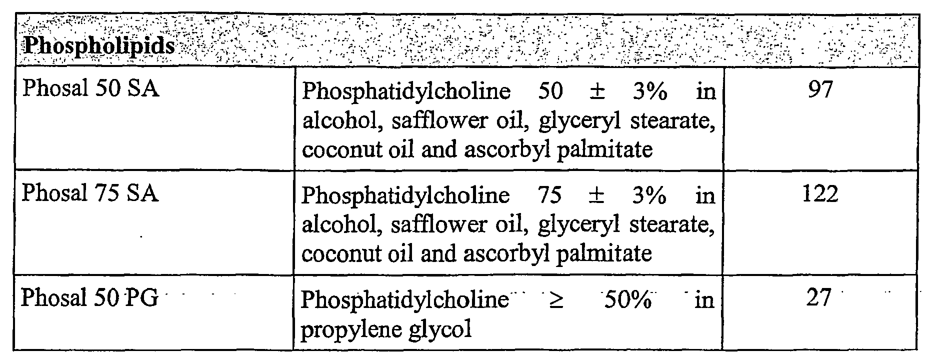

The following table 2 reports the chemical composition of the selected excipients in semi-solid form at room temperature, as well as the solubility of a taxoid of formula Ib. Excipients had been previously melted up to 70°C for drug dissolution.

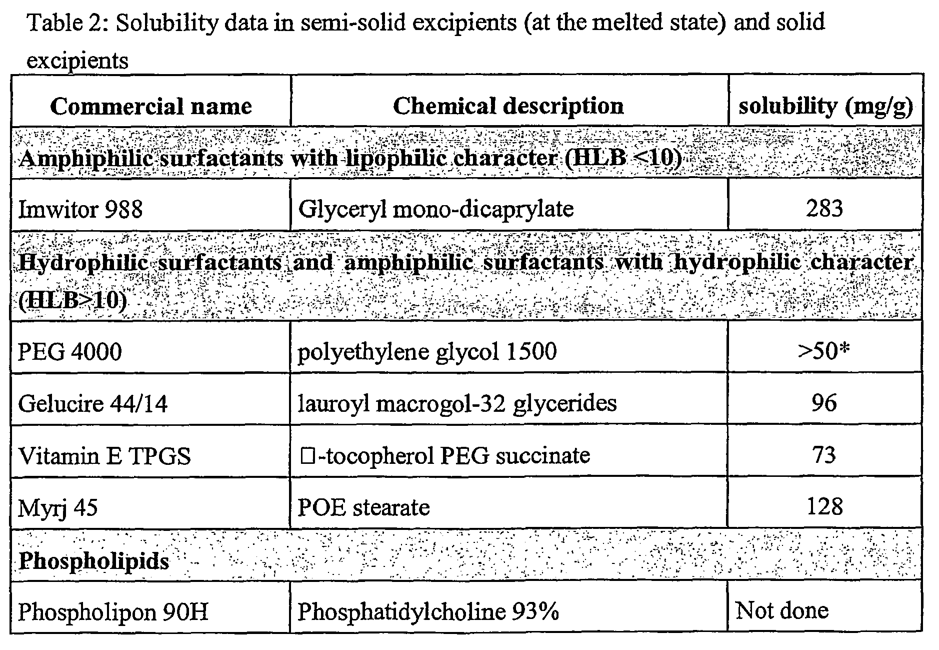

The solubility of taxoid of formula Ib at room temperature has been determined by X ray diffraction.
Taking into account the solubility of a taxoid of formula Ib, for the 3 categories of drug delivery systems the following excipients were retained:
Vitamin E TPGS for micelle formation
Phosal 75SA and Phospholipon 90H for lipidic vesicle formation
Labrasol and Gelucire 44/14 for emulsion formation
Microemulsion formation: as surfactant Myrj 45, PS80, Cremophor EL, Labrasol; as co-surfactant: Maisine, Capryol 90, Peceol, Lauroglycol 90, Imwitor 988; as oil: Miglyol 812N, Edenor.

For the first 3 categories, the excipients were formulated as binary systems with the drug, at the following concentrations:
- Vitamin E TPGS (semi-solid matrix): 50, 100 mg/g formulation
- Phosal 75SA (solution): 100 mg/g formulation
- Phospholipon 90H (solid powder): 50, 100 mg/g formulation
- Gelucire 44/14 (semi-solid matrix): 50, 100 mg/g formulation
- Labrasol (solution): 50, 100, 200 mg/g formulation

For the SMES category (3-components system), a first screening of the excipients as oil, surfactant (HLB>10) and co-surfactant (HLB<10), combined together at different ratios without the presence of the active, was necessary for identifying the formulations able to form a microemulsion (droplet size <30 nm) after infinite dilution with water. With this screening the following SMES were identified:
- Cremophor EL/Maisine/Miglyol 812N at 50 mg/g
- Cremophor EL/Lauroglycol 90/Miglyol 812N at 50 mg/g
- Cremophor EL/Capryol 90/Miglyol 812N at 50 mg/g
- Cremophor EL/Peceol/Miglyol 812N at 50 mg/g
- Cremophor EL/Imwitor 988/Miglyol 812N at 50 mg/g

The ratio between the excipients in the retained formulations was as follow: ratio surfactant to co-surfactant 3:1 and with oil concentration of 20%.

It is understood that the dosage may vary according to the degree or the nature of the condition to be treated. Thus, the quantity of active product in a composition according to the invention will be determined such that a suitable dosage can be prescribed. As a result, the quantity of taxoids varies as a function of its solubility in the mixture and also as a function of the appropriate dosage for the treatment of patients. Preferably, care should be taken not to load more than 10% w/w of taxoid drug so as to avoid microemulsion destabilization to occur.

In humans, it is understood that, to choose the most appropriate daily dosage, there should be taken into account the weight of the patient, his general state of health, his age and all factors which may influence the efficacy of the treatment. Preferably, the compositions are prepared such that a unit dose contains from 0.1 to 50 mg of active product.

According to the invention, the pharmaceutical composition may be obtained by mixing, where appropriate, the principal excipient (after heating for melting the semisolid excipient), followed by the addition of the taxoid and maintaining stirred in order to obtain a homogeneous mixture.

The compositions according to the invention may be provided in the semipasty state.

They are particularly suitable for presentation in the form of hard gelatin capsules or soft gelatin capsules, or in the form of an oral solution.

The compositions according to the invention are particularly advantageous because of their good stability, both physically and chemically, and the enhancement of the bioavailablity which they offer upon oral administration of taxoids.

The following examples, given without limitation, illustrate formulations according to the present invention.

### FIGURES

Figure 1 : Taxoid of formula Ib relase profile of different formulations at 100 mg/g in simulated gastric medium
Figure 2 : Taxoid of formula Ib relase profile of semi-solid formulations at 50 and 100 mg/g in simulated gastric medium
Figure 3 : Particle size of Taxoid of formula Ib formulations in simulated gastric medium
Figure 4 : Particle size of Taxoid of formula Ib formulations leading to droplets <50 nm in simulated gastric medium

### EXAMPLES

### Example 1 : Preparation of Prototypes

### 1.1 Materials

- Taxoid of formula Ib
- Miglyol 812N (Condea Vista Company, Cranford, NJ, USA)
- Labrasol (Gattefossé, Saint Priest, F)
- Gelucire 44/14 (Gattefossé, Saint Priest, F)
- Vitamin E TPGS (Eastman Chemical, Anglesey, UK)
- Cremophor EL (BASF AG, Ludwigshafen, DE)
- Capryol 90 (Gattefossé, Saint Priest, F)
- Lauroglycol 90 (Gattefossé, Saint Priest, F)
- Peceol (Gattefossé, Saint Priest, F)
- Maisine 35-1 (Gattefossé, Saint Priest, F)
- Imwitor 988 (Condea Vista Company, Cranford, NJ, USA)
- Phosal 75SA (Nattermann, Cologne, DE)
- Phospholipon 90H (Nattermann, Cologne, DE)
- PS80 VG DF (Seppic, Paris, France)

### 1.2 Preparation of the semi-solid matrices

The weighed drug was dispersed in the melted excipient, and then maintained under mechanical stirring at 50-60°C until dissolution. The mass was poured into a hard gelatine capsule (size 0) and kept refrigerated overnight. The gelatine shell was then removed to avoid compatibility issues at this step.

### 1.3 Chemical stability

The chemical stability of the different formulations is a key parameter. Prototypes were stored in bulk (glass vial) for up to 3 months at +5°C (± 3°C), 25°C (± 2°C) and 30°C (± 2°C) under 60% (± 5%) relative humidity (RH) and 40°C (± 2°C) under 75% (± 5%) RH.
The stability was evaluated by mean of the potency determined by HPLC, as well as evaluation of relative substances. The prototypes analysed for drug dosage and stability studies are showed in the table below.

**Table 3: Prototypes of taxoid of formula Ib formulations for stability study**

| **PROTOTYPE** | **DRUG CONCENTRATION mg/g formulation** |
|---|---|
| PS 80 | 100 |
| Capryol 90 | 250 |
| Labrasol | 100 |
| Labrasol | 200 |
| Phosal 75 SA | 100 |
| Gelucire 44/14 | 80 |
| Gelucire 44/14 | 100 |
| Vitamin E-TPGS | 60 |
| Vitamin E-TPGS | 100 |
| CremophorEL-Miglyol 812N-Peceol | 50 |
| CremophorEL-Miglyol 812N-Maisine | 50 |
| CremophorEL-Miglyol 812N-Lauroglycol 90 | 50 |
| Cremophor EL-Miglyol 812N-Capryol 90 | 50 |
| Cremophor EL-Miglyol 812N-Imwitor 988 | 50 |
| Phospholipon 90 H | 50 |
| Phospholipon 90 H | 100 |

All the formulations are stable 3 months at 40°C under 75% RH, except the SMES formulations. Indeed, the SMES are stable 1 month at 25°C, whereas at 40°C the impurity taxoid of formula Ib (hydrolysis) appears (1.15-3.88% at t_{1 month}, depending on the nature of the co-surfactant). The 3 months analysis of the sample allowed to evaluate if this impurity increase was critical: after 3 months, an increase of taxoid of formula Ib impurity content was noticed. The SMES is stable at 5°C during 7 months.

### Example 2 : in vitro behaviour in simulated gi media (gi = gastro intestinal)

### Release profiles after incubation in simulated gi media

### 2.1 Composition of the simulated fluids

The following simulated media were selected for the present experiment:
- Gastric medium USP, pH 1.2
- Fasted intestinal medium, pH 6.8 (ref. Dressman et al., Pharm. Res., 1998)
- Fed intestinal medium, pH 5 (ref. Dressman et al., Pharm. Res., 1998)

**Table 4: Composition of the simulated gastro-intestinal media**

| **Gastric medium (G)** | | |
|---|---|---|
| Sodium chloride | 2 g | |
| Hydrogen chloride 1N | 100 ml approximately | |
| Demineralised water | qsp 1000 ml | |
| | | |

| **Fasted intestinal medium (Fassif)** | | For 500 ml |
|---|---|---|
| Potassium hydrogenophosphate | 0.029 M | 1.97 g |
| Sodium hydroxide | qs pH 6.8 | qs pH 6.8 |
| Sodium Taurocholate | 5 mM | 1.34 g |
| Lecithin (Phospholipon 90G) | 1.5 mM | 0.58 g |
| Potassium chloride | 0.22 M | 8.2 g |
| Demineralised water | qsp 11 | qsp 500 ml |
| | | |

| **Fed intestinal medium (Fessif)** | | For 500 ml |
|---|---|---|
| Acetic acid Sodium hydroxide | 0.144M qs pH 5 | 4.33 g qs pH 5 |
| Sodium Taurocholate | 15 mM | 4.03 g |
| Lecithin (Phospholipon 90G) | 4 mM | 1.55 g |
| Potassium chloride | 0.19 M | 7.08 g |
| Demineralised water | qsp 11 | qsp 500 ml |

### 2.2 Experimental conditions

In a first step of experiments, the formulations (100 mg drug/g formulation, 500 mg formulation in a hard gelatin capsule) were diluted 1:500 in the gastric medium (1 capsule/250 mL), than incubated 2 hours at 37°C under stirring (50 rpm) in a USP standard dissolution apparatus.
The same experiment has been carried out in gastric medium with 2 capsules loaded with less concentrated formulations (50 mg drug/g formulation), in order to study the effect of the drug/excipient and excipient/medium ratio on the release profile.
In a second step of experiments, a first incubation of 1 hour in gastric medium was followed by 2 hours incubation in fasted intestinal or fed intestinal medium, in order to simulate the gastric emptying process.

Samples were taken after 5-15-30-60 min and 2h. The drug concentration was determined by HPLC after centrifugation (6000 rpm, 10 min). Homogeneity of the medium was evaluated by sampling bottom, medium and top of the vessel.

### 2.3 Results

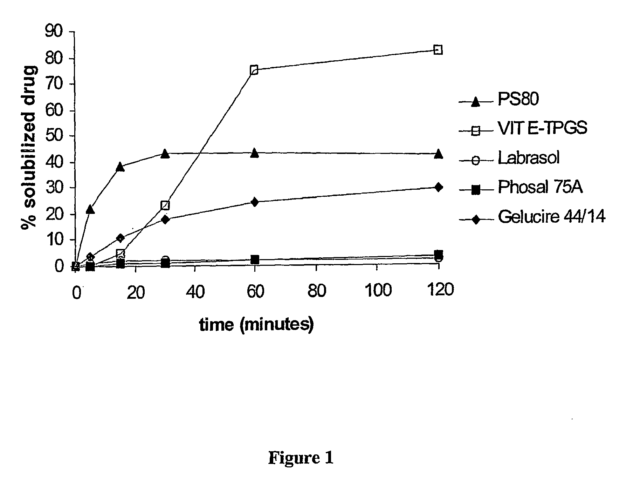

Drug release profiles in gastric medium of formulations at 100 mg/g are shown in the figure 1.
Compared to the PS80 formulation (evaluated as reference), only the formulation composed of Vitamin E TPGS allowed improvement of the *in vitro* solubilisation of taxoid of formula Ib (80% of drug solubilised) by 2 hours.
Concerning the profiles obtained with the other formulations data from Phosal and Gelucire are not very representative, since these formulations led to the formation of a very heterogeneous mixture after incubation. For Gélucire 44/14, the disintegration of the semi-slid matrix occurred only partially, not allowing the dispersion in the simulated gastric medium. The Labrasol formulation led to the formation of a very homogeneous emulsion with the medium, despite the low amount of drug recovered after centrifugation (see release profile), suggesting that for a coarse emulsion the centrifugation (determining the collapse of the emulsion) could sub-estimate its *in vitro* performance. The experiment with Phospholipon 90H was stopped (no data collection) since the powder floating did not allow the formation of a homogeneous suspension.

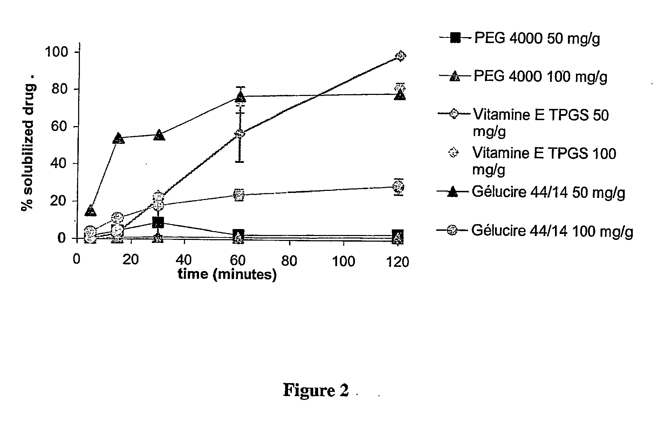

The comparison of the drug relase profiles of semi-solid formulations (Gelucire, Vitamin E TPGS and PEG 4000) at 50 et 100 mg/g (figure 2; the profiles related to Vitamin E TPGS and Gelucire at 100 mg/g are the same already reported in figure 1) shows that Vitamin E TPGS exhibited the highest solubilisation properties, with a release of 80% for the 100 mg/g dosage and up to 100% for the 50 mg/g dosage. The Gelucire formulation at 50 mg/g allowed the solubilisation of about 80% of drug, contrarily to the 100 mg/g dosage, as described previously. Finally, the hydrophilic PEG 4000 confirmed, as expected, the inability to solubilise a hydrophobic drug in an aqueous medium.

### Example 3 : particle size analysis after incubation in gastric medium (USP)

The aim of this part of the study was to evaluate, by particle size measurement, the colloidal stability and the self-emulsifying properties of the emulsion/microemulsion/micellar solution of taxoid of formula Ib formulations after incubation in the gastric medium.

### 3.1 Experimental conditions

The formulations (concentration 100 mg drug/g formulation, 100 mg formulation) were diluted 1:500 in the gastric medium (50 mL), then incubated 2 hours at 37°C under mechanical stirring (300 rpm).
The sample was diluted immediately with water for size measurement or filtered onto 2 µm if necessary. The filtration allowed to retain oil droplets>2 µm, as well as drug crystals >2 µm, in order to allow the particle size measurement by QELS (quasi-elastic light scattering) (Nanosizer N4+, Beckmann-Coulter).

### 3.2 Results

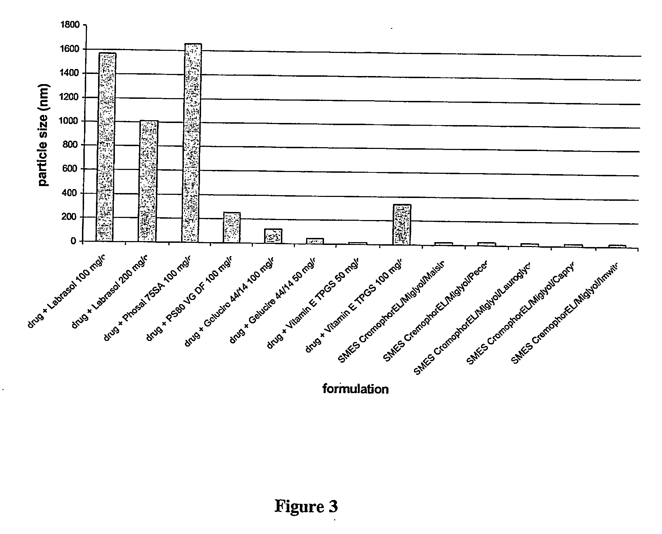

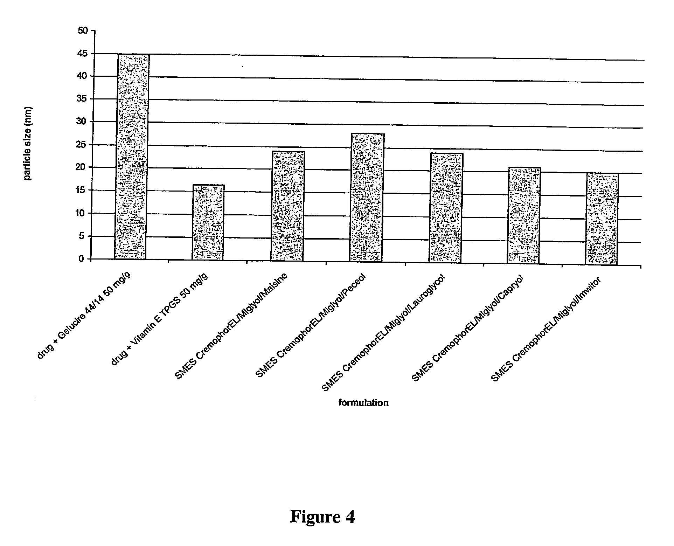

As shown in the figures 3 and 4, a particle size <50 nm was obtained only in the case of the formulations with active concentration of 50 mg/g: the 5 microemulsions (nevertheless their composition), Gelucire (after 2 µm filtration) and Vitamin E TPGS.

The results suggest using the formulations able to form small and monodisperse droplets in gastric medium in order to have a better performance *in vivo.* Further experiments in simulated intestinal media should be performed in order to evaluate the effect of biliary salts on the size and colloidal stability of the formulations.

### 3.3 Preliminary conclusions on the evaluation of taxoid of formula Ib formulations

All the results concerning the *in vitro* behaviour in simulated GI fluids of the formulations for oral administration of taxoid of formula Ib, as well as the chemical stability in accelerated conditions, are summarized in the tables below.

**Table 5: Summary of the in vitro behaviour of the formulations at 50 mg/g**

| Formulation | Chemical Stability | Droplet size *in vitro* (2h at 37°C in gastric medium) | Homogeneity *in vitro* (2h at 37°C in gastric medium) | % released drug *in vitro* after 2h in gastric medium) | % released drug *in vitro* after 1h gastric + 2h Fassif |
|---|---|---|---|---|---|
| Vitamin E TPGS | >3 months at 40°C/75%RH | 16 nm | Good | 100% | Not done |
| Gélucire 44/14 | >3 months at 40°C/75%RH | 45 nm (after filtration) | Good | 79% | >60% |

**Table 6: Summary of the in vitro behaviour of the formulations at 100 mg/g**

| Formulation | Chemical Stability | Droplet size *in vitro* (2h at 37°C in gastric medium) | Homogeneity *in vitro* (2h at 37°C in gastric medium) | % released drug *in vitro* after 2h in gastric medium) | % released drug *in vitro* after 1h gastric + 2h Fassif |
|---|---|---|---|---|---|
| Vitamin E TPGS | >3 months at 40°C/75%RH | 335 nm | Good | 82-84% | 60% |
| Gélucire 44/14 | >3 months at 40°C/75%RH | 118 nm (after filtration) | Bad | Not determined | Not determined |

Gelucire leads to a heterogeneous emulsion with the GI media, so it was discarded at this concentration. Thus, at 100 mg/g, only Vitamin E TPGS formulation exhibited a promising behaviour (in terms of release profile and droplet size).

### Example 4 : Physical characterization and stability of semi-solid matrices

### 4.1 Experimental methods

### X-RAY POWDER DIFFRACTION (XRPD)

The analyses are carried out on a Siemens-Bruker D5000 Matic diffractometer, using the parafocusing Bragg-Brentano (*θ*-2*θ*)- type geometry. If enough of the product is available, the powder is deposited on a concave aluminum sample holder. Otherwise a thin layer of the product is deposited on a single-crystalline silicon wafer, cut out according to the (510) crystallographic orientation that impedes any Bragg reflection (by ensuring the systematic extinction of the corresponding diffraction band). A cobalt anticathode tube (40kV/30mA) gives an iron-filtered incident beam. Two radiations are emitted: Co*K*α₁ (λ = 1.7890 Å) and Co*Kα₂* (*λ* = 1.7929 Å. A 50 M multicanal Braun linear detector completes the setup. It has a 10°-wide detection window in angle 2*θ*. Diagrams were recorded in the following conditions: a 1.5 to 50.0 degree scan in angle 2*θ*, 10 to 30 seconds' counting time per degree in 2θ according to the amount of powder to be analysed, and ambient conditions of pressure, temperature and % relative humidity.

### 4.1.2 Physical characterization

Taxoid of formula Ib semi-solid formulations for this part of the study were manufactured and characterized by T.Borovac (DEA report «Conception et caractérisation des matrices semi-solides associées à un principe actif peu hydrosoluble et destiné à la voie orale», CRS meeting, July 19, 2003.
In the semi-solid formulations, drug substance physical state (solubilized or dispersed) and physical form (if dispersed) were characterized using XRPD. This technique detection limit was evaluated using a range of physical mixtures (of Vitamin E-TPGS or Gelucire and drug substance): this limit is 2.5% or 25 mg/g with both excipients.

### 4.2 Physical stability

Storage conditions (temperature, pressure, time) can change or induce recrystallization of drug substance in a solubilized semi-solid formulation or polymorphism in a dispersed one.
Two semi-solid formulations (a 60 mg/g VitaminE-TPGS one and a 80 mg/g Gelucire one) were evaluated after one month at 30°C/60%RH or at 40°C/75%RH. Both formulations were mostly solubilised after manufacturing and no recrystallisation was observed after one month. We can anticipate that both 50 mg/g formulations are physically stable for at least one month.

### 4.3 Conclusions and further studies

**Table 7: Comparative properties of the recommended formulations according to the selection criteria**

| | **FORMULATION** | |
|---|---|---|
| **Criteria** | **TPGS** | **Gélucire** |
| Safety excipients | Yes | Yes |
| Registrability excipients | Yes | Yes |
| Developpability/registrability formulation | Yes | Yes |
| Conc.>50 mg/g | Yes (up to 100) | Yes (up to 50) |
| Chemical Stability t_{3 months} | 40°C/75%RH | 40°C/75%RH |
| Solubilisation in GI media | Very good | Very good |
| Fine droplet size (GI media) | Yes (16 nm) | Yes (45 nm) |
| Physical Stability t_{1 months} | 40°C/75%RH | 40°C/75%RH |

## Claims

1. Semi-solid formulation for the oral administration of taxoids consisting of binary system of one taxoid and Vitamin E TPGS®.

2. Semi-solid formulation according to claim 1, wherein the taxoid is a taxoid of general formula (I): 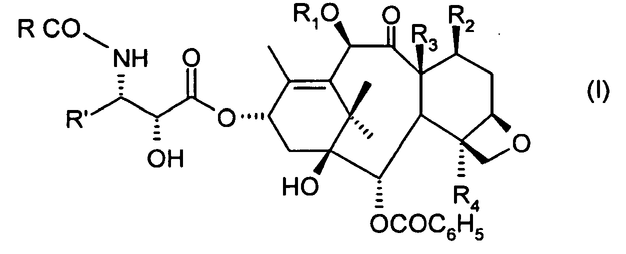 wherein :
R₁ is H, acyl (C₂-C₄), alkyl (C₁-C₃);
R₂ is OH, alkoxy or R₂ and R₃ are methylene;
R₃ is CH₃ or R₂ and R₃ are methylene;
R₄ is OCOCH₃ or OCOOCH₃;
R is phenyl or alkoxy (C₃-C₄) or alkenyloxy (C₃-C₄), preferably phenyl or tert-butoxy; and
R' is aryl, preferably phenyl, optionally substituted or alkyl (C₂-C₄) or alkylen (C₂-C₄).

3. Semi-solid formulation according to claim 2, wherein the taxoid is chosen among compounds of formula (Ia) to (If):

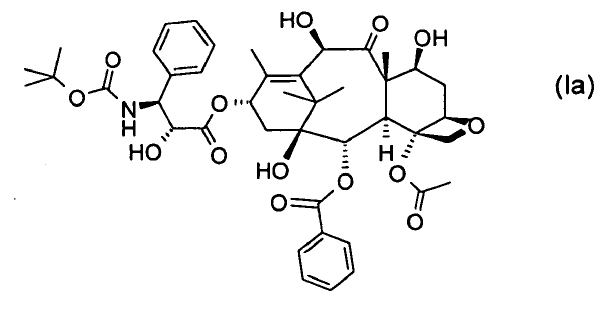

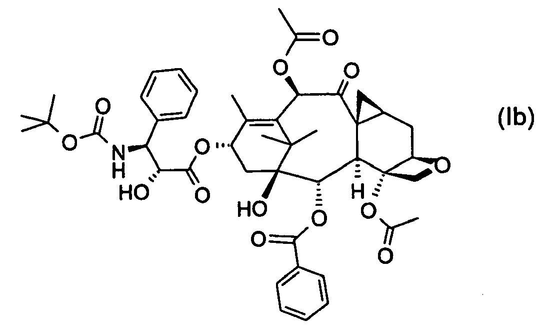

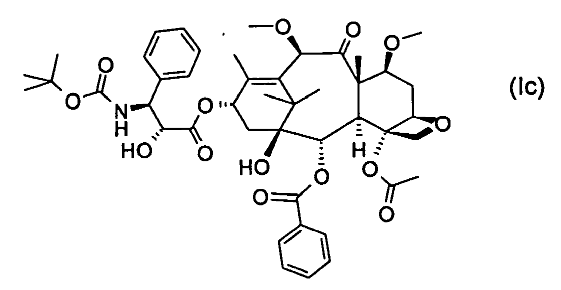

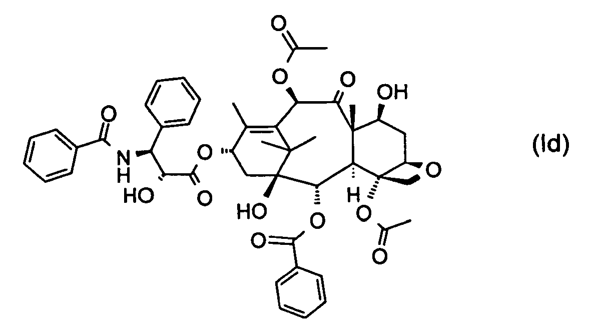

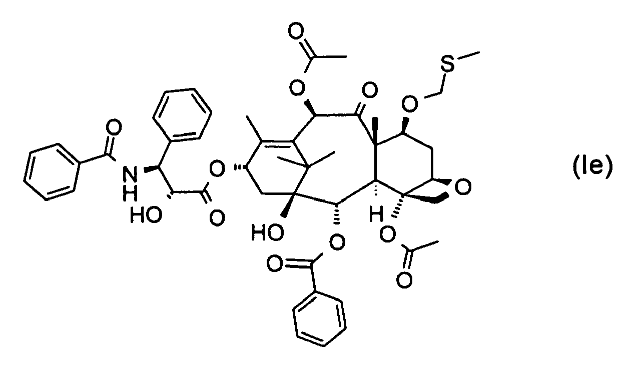

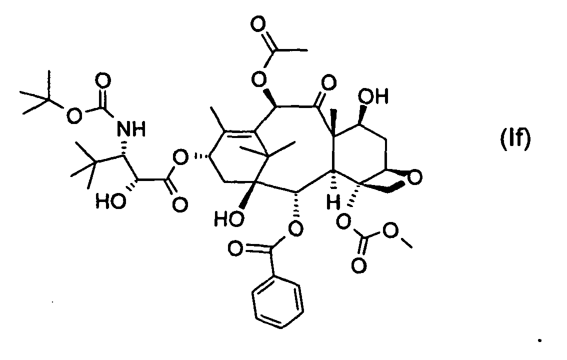

4. Semi-solid formulation according to claim 3, wherein the taxoid is chosen among compounds of formula (Ib) and (Ic).

5. Semi-solid formulation as claimed in claims 1-4, wherein the formulation contains up to 200 mg taxoid per g of polymeric material.

6. Semi-solid formulation as claimed in claim 5, wherein the formulation contains between 5 and 100 mg taxoid per g of polymeric material.

7. A process for preparing a formulation as claimed in claims 1 to 6, wherein there is prepared, where appropriate, the principal excipient, after heating, for melting the semisolid excipient, and then the taxoid and stirring is maintained in order to obtain a homogeneous mixture.

## Patentansprüche

1. Halbfeste Formulierung zur oralen Verabreichung von Taxoiden, umfassend ein binäres System aus einem Taxoid und Vitamin E TPGS®.

2. Halbfeste Formulierung gemäß Anspruch 1, wobei das Taxoid ein Taxoid der allgemeinen Formel (I) ist, 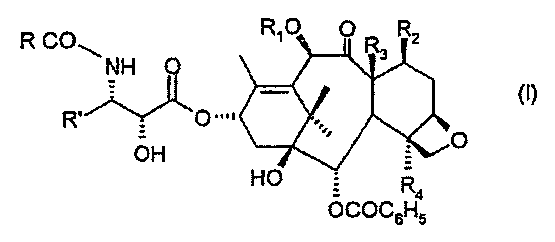 wobei:
R₁ H, Acyl (C₂-C₄), Alkyl (C₁-C₃) ist;
R₂ OH, Alkoxy ist, oder R₂ und R₃ Methylen sind;
R₃ CH₃ ist, oder R₂ und R₃ Methylen sind;
R₄ OCOCH₃ oder OCOOCH₃ ist;
R Phenyl oder Alkoxy (C₃-C₄) oder Alkenyloxy (C₃-C₄) ist, vorzugsweise Phenyl oder *tert*-Butoxy; und
R' Aryl ist, vorzugsweise Phenyl, das gegebenenfalls substituiert ist, oder Alkyl (C₂-C₄) oder Alkylen (C₂-C₄).

3. Halbfeste Formulierung gemäß Anspruch 2, wobei das Taxoid aus Verbindungen der Formeln (Ia) bis (If) ausgewählt ist:

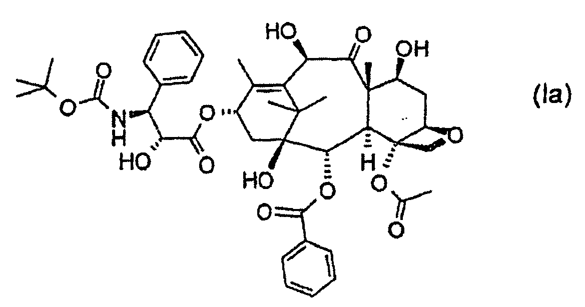

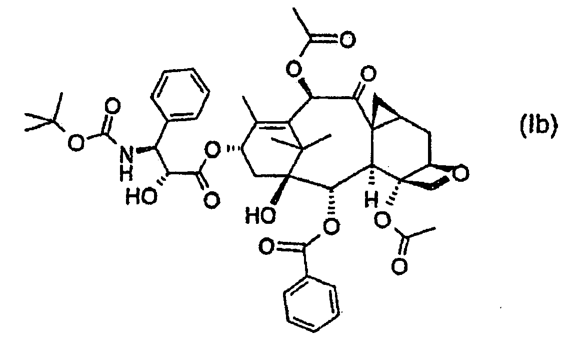

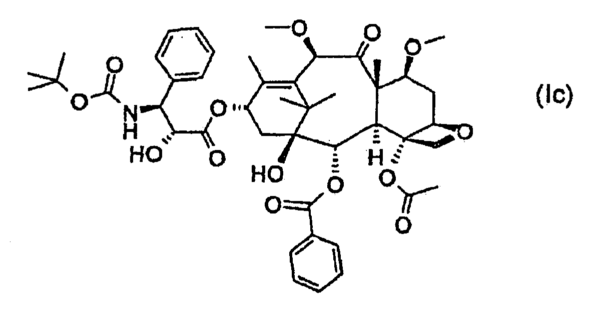

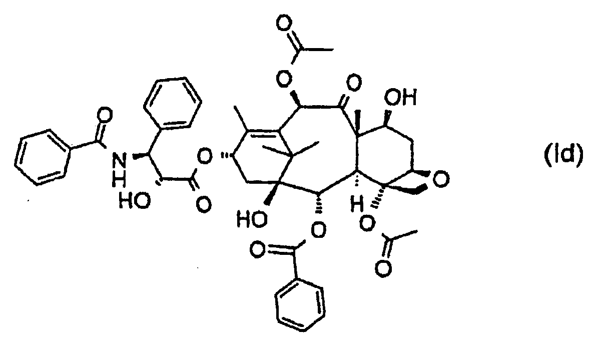

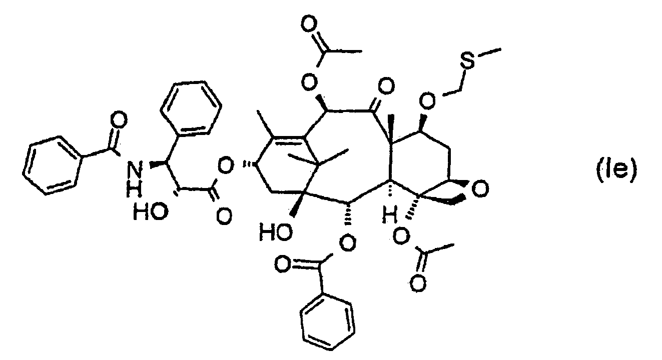

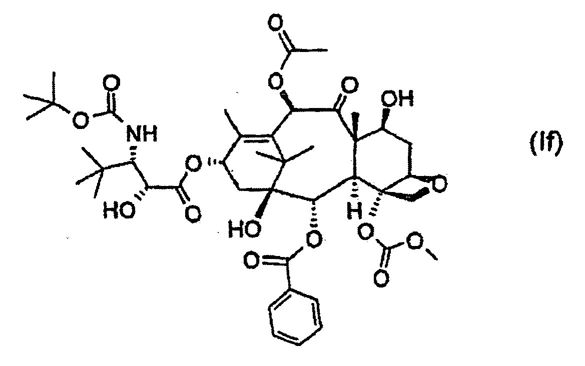

4. Halbfeste Formulierung gemäß Anspruch 3, wobei das Taxoid aus Verbindungen der Formeln (Ib) und (Ic) ausgewählt ist.

5. Halbfeste Formulierung gemäß Ansprüchen 1-4, wobei die Formulierung bis zu 200 mg Taxoid pro g Polymermaterial umfasst.

6. Halbfeste Formulierung gemäß Anspruch 5, wobei die Formulierung zwischen 5 und 100 mg Taxoid pro g Polymermaterial umfasst.

7. Verfahren zum Herstellen einer Formulierung gemäß Ansprüchen 1 bis 6, wobei, wenn geeignet, der Hauptexzipient nach Erwärmen zum Schmelzen des halbfesten Exzipienten und anschließend das Taxoid angesetzt wird, wobei anhaltend gerührt wird, um ein homogenes Gemisch zu erhalten.

## Revendications

1. Formulation semi-solide pour l'administration orale de taxoïdes constituée d'un système binaire d'un taxoïde et de vitamine E TPGS®.

2. Formulation semi-solide selon la revendication 1, dans laquelle le taxoïde est un taxoïde de formule générale (I) : 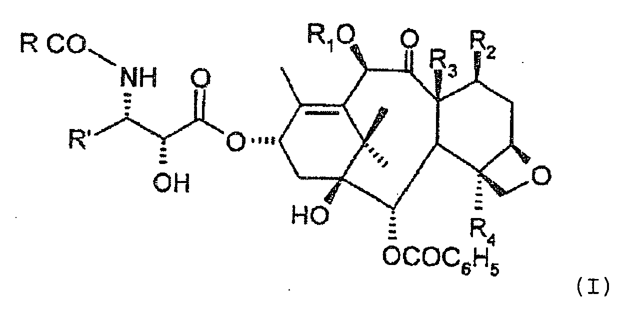 dans laquelle :
R₁ est H, un acyle en C₂-C₄, un alkyle en C₁-C₃ ;
R₂ est OH, un alcoxy ou R₂ et R₃ sont un méthylène ;
R₃ est CH₃ ou R₂ et R₃ sont un méthylène ;
R₄ est OCOCH₃ ou OCOOCH₃ ;
R est un phényle ou un alcoxy en C₃-C₄ ou un alcényloxy en C₃-C₄, de préférence un phényle ou un tert-butoxy ; et
R' est un aryle, de préférence un phényle, facultativement substitué ou un alkyle en C₂-C₄ ou un alkylène en C₂-C₄.

3. Formulation semi-solide selon la revendication 2, dans laquelle le taxoïde est choisi parmi des composés de formule (Ia) à (If) :

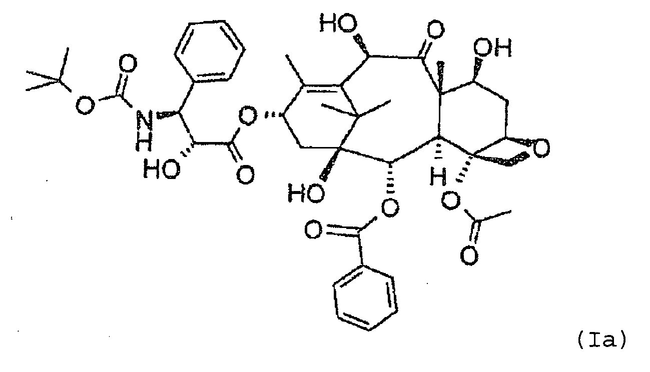

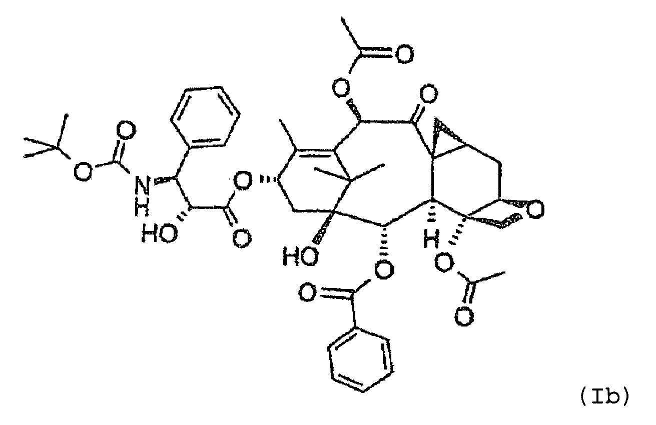

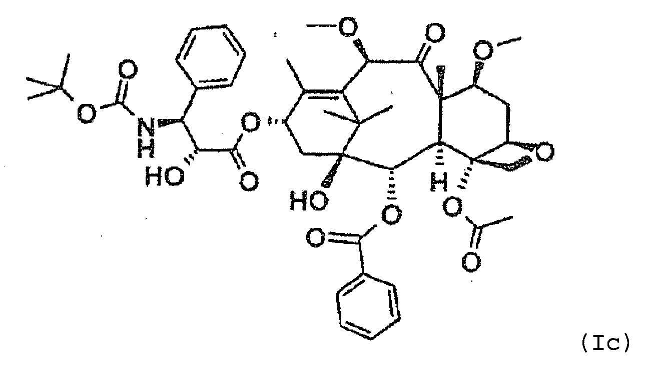

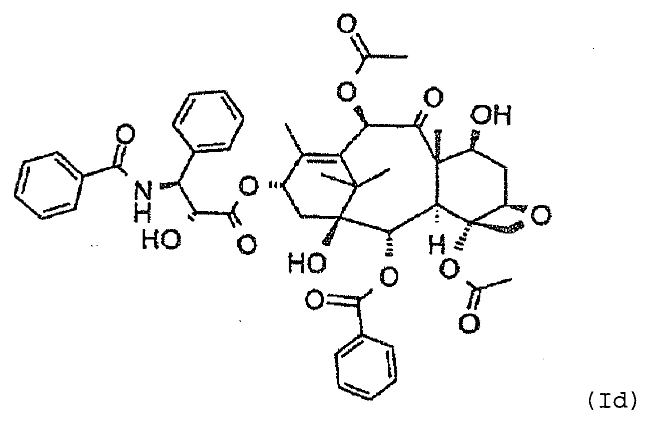

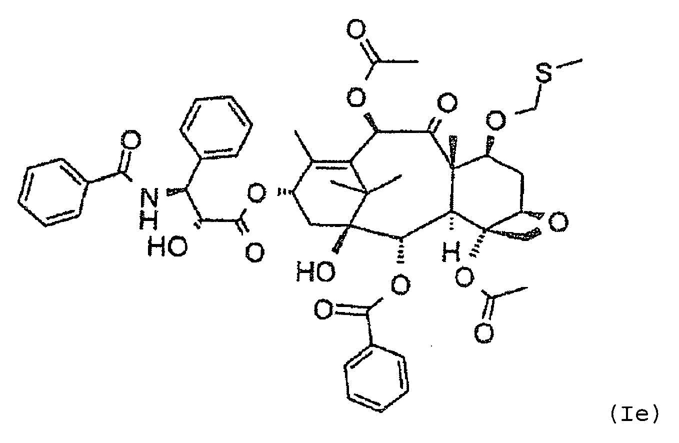

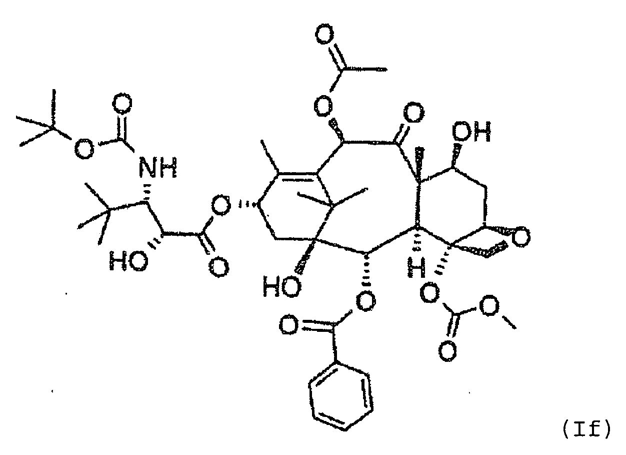

4. Formulation semi-solide selon la revendication 3, dans laquelle le taxoïde est choisi parmi des composés de formule (Ib) et (Ic).

5. Formulation semi-solide selon les revendications 1 à 4, dans laquelle la formulation contient jusqu'à 200 mg de taxoïde par g de matériau polymère.

6. Formulation semi-solide selon la revendication 5, dans laquelle la formulation contient entre 5 et 100 mg de taxoïde par g de matériau polymère.

7. Procédé de préparation d'une formulation selon les revendications 1 à 6, dans lequel il est préparé, de manière appropriée, l'excipient principal, après chauffage, pour faire fondre l'excipient semi-solide, et ensuite le taxoïde et l'agitation est maintenue afin d'obtenir un mélange homogène.
